# EUROPÄISCHE PATENTANMELDUNG

(11) **EP 3 281 672 A1**
(43) Veröffentlichungstag der Anmeldung: **14.02.2018**
(21) Anmeldenummer: 16183310.8
(22) Anmeldetag: 09.08.2016
(51) Int. Cl.: A61N 5/06, A61L 2/00, A61L 2/025, A61L 2/14, A61L 2/10, A61M 1/06, A41C 3/04

(54) **TRAGBARE VORRICHTUNG ZUR DEKONTAMINATION EINER BRUST**

(71) Anmelder: Carag AG, 6340 Baar (CH)
(72) Erfinder: Hartmann, Peter, Gooseberry Hill WA 6076 (AU); Larsson, Michael, 6300 Zug (CH); Limacher, Kuno, 6312 Steinhausen (CH); Bernhard, Jerome, 8003 Zürich (CH); Christen, Lukas, 6004 Luzern (CH); Wiedmer, Simona, 6300 Zug (CH); Napoletano, Daniel, 8455 Rüdlingen (CH)
(74) Vertreter: Grünecker Patent- und Rechtsanwälte PartG mbB

(57) **Zusammenfassung**

Die vorliegende Erfindung betrifft eine tragbare Vorrichtung zur Dekontamination einer Brust (2). Mittels der Vorrichtung soll die Gefahr einer Mastitis reduziert werden, auch wenn bei dem Patienten eine Medikamentenunverträglichkeit besteht. Hierzu schlägt die Erfindung eine Dekontaminationseinheit (9) vor, welche die Brust (2) zumindest mittels einer physikalischen Methode dekontaminiert.

## Beschreibung

Die vorliegende Erfindung betrifft eine tragbare Vorrichtung zur Dekontamination einer Brust.

Eine gattungsgemäße Vorrichtung mit den oberbegrifflichen Merkmalen von Anspruch 1 ist beispielsweise aus der US 2003/0073930 A1 bekannt. Die in diesem Dokument beschriebene Vorrichtung weist eine Glocke auf, die über eine weibliche Brust eines Menschen gestülpt wird. Innerhalb der Glocke ist ein Druckelement vorgesehen auf dessen Oberfläche ein absorbierendes Material angebracht ist. Durch den von dem Druckelement aufgebrachten Druck wird das absorbierende Material gegen die Brustwarze gedrückt um entweder Feuchtigkeit von dieser aufzunehmen oder aber ein medizinisches Reagenz, wie beispielsweise ein fungizides oder antibakterielles Reagenz aufzutragen. So wird beispielsweise eine Entzündung der Brust geheilt bzw. reduziert.

Bei der laktierenden Brust einer Mutter besteht eine erhöhte Gefahr einer Mastitis. Grund dafür ist, dass nach Entnahme von Milch, zum Beispiel durch Abpumpen oder durch den Säugling, die Milchgänge noch geöffnet sind, was zu einer Invasion von Krankheitserregern führen kann.

Zwar kann das aus der US 2003/0073930 A1 bekannte System die Gefahr einer Mastitis reduzieren, es kann jedoch vorkommen, dass der betreffende Patient das Medikament mit welchem das absorbierende Material getränkt ist, nicht verträgt.

Im Lichte dieser Problematik schlägt die vorliegende Erfindung eine tragbare Vorrichtung zur Dekontamination einer Brust mit den Merkmalen von Anspruch 1 vor.

Die tragbare Dekontaminationsvorrichtung zeichnet sich insbesondere dadurch aus, dass die Dekontaminationseinheit die Brust mittels einer physikalischen Methode dekontaminiert.

Dadurch dass eine solche physikalische Methode verwendet wird, braucht keine flüssige oder pastöse Substanz zur Dekontamination verwendet werden. Im Gegensatz zu biologischen oder chemischen Dekontaminationsverfahren wird vorliegend zumindest eine physikalische Dekontamination durchgeführt. Eine physikalische Größe wird demnach als Mittel der Kontamination verwendet. Diese Größe wird zum Beispiel mittels elektrischer Energie erzeugt und die Brust wird der mittels elektrischer Energie erzeugten Größe ausgesetzt.

Vorteilhaft bei einer solchen physikalischen Dekontamination ist, dass diese nicht lediglich gegen einzelne Spezies von Mikroorganismen wirkt, sondern "breitbandig" gegen eine Vielzahl von Mikroorganismen. Der Begriff "Mikroorganismus" wird in diesem Zusammenhang wie folgt verwendet. Mikroorganismen können mikroskopisch kleine Lebewesen (Organismen) sein, die als Einzelwesen nicht mit bloßem Auge erkennbar sind. Sie werden auch als Mikroben oder Kleinstlebewesen bezeichnet. Sie bilden im System der Lebewesen keine einheitliche Gruppe. Zu den Mikroorganismen zählen Bakterien (z. B. Milchsäurebakterien), Pilze (z. B. Backhefe), mikroskopische Algen (z. B. Chlorellen) sowie Protozoen (z. B. Pantoffeltierchen und der Malaria-Erreger Plasmodium). Vorliegend werden auch Viren zu den Mikroorganismen gerechnet. Viren werden zwar überwiegend nicht als Lebewesen, also auch nicht als Mikroorganismen angesehen, gelegentlich werden sie aber dennoch zu den Mikroorganismen gezählt und dann wird dementsprechend die Virenforschung (Virologie) als ein Teilgebiet der Mikrobiologie angesehen.

Zudem bringt eine solche physikalische Dekontamination den Vorteil, dass keine Resistenzen auftreten, wie es der Fall ist, wenn z.B. auf Antibiotika (chemische Reagenzien) zur Dekontamination verwendet werden.

Auch ist die Behandlung während der Zeit des Säugens von Kindern möglich, da die Brustwarze rückstandsfrei, für die Kinder zur Nahrungsaufnahme zur Verfügung steht.

Gemäß einer vorteilhaften Weiterbildung der Erfindung kann diese Dekontaminationseinheit zumindest eines der folgenden Elemente enthalten: eine Lichtquelle, insbesondere UV-Lichtquelle, eine Ultraschallquelle, eine Ozonisierungsquelle, eine Plasma-, insbesondere Kaltplasmaquelle. Diese Quellen erzeugen alle energetische/hochenergetische Strahlung und/oder energetische/hochenergetische Teilchen und/oder energetische/hochenergetische Moleküle und/oder energetische/hochenergetische Wellen, welche zur Dekontamination mit entsprechenden Erregern, wie Bakterien und Viren, wechselwirken, um diese unschädlich zu machen. Zur Erzeugung dieser Strahlung bzw. Teilchen wird z.B. eine Energiequelle, insbesondere elektrische Energiequelle verwendet.

Als Lichtquelle kann eine LED insbesondere eine UV-LED Verwendung finden. Als Ultraschallquelle kann beispielsweise ein piezokeramisches Element Verwendung finden. Beide zuvor genannten Elemente können heutzutage sehr klein gebaut werden und können in unmittelbarer Nähe der zu dekontaminierenden Stelle der Brust montiert werden.

Gemäß einer vorteilhaften Weiterbildung der Erfindung kann die Vorrichtung eine glockenartige Struktur aufweisen, welche über die Brust stülpbar ist und diese zumindest bereichsweise umstülpt. Diese glockenartige Struktur kann demnach über die Brust gestülpt werden. Die glockenartige Struktur kann in diesem Fall schon so vorgeformt sein, dass diese an die Brust anlegbar ist. Innerhalb dieser glockenartigen Struktur kann dann die entsprechende Dekontaminationseinheit vorgesehen sein. Durch die glockenartige Struktur wird gewährleistet, dass der Bereich der Brust der dekontaminiert werden soll, von der äußeren Umgebungsatmosphäre abgeschlossen ist und der Bereich der Brustoberfläche innerhalb der Glocke effizient dekontaminiert wird. Diese glockenartige Struktur kann eine Art Kappe sein, welche über die Brust gestülpt wird.

Gemäß einer vorteilhaften Weiterbildung der Erfindung kann die glockenartige Struktur so ausgebildet sein, das sich diese erst bei Anlegen an die Brust instantan ausbildet und vorzugsweise bei Entfernung von der Brust wieder in deren Grundzustand zurückkehrt. Die glockenartige Struktur kann auch so ausgebildet sein, das sie in einem nicht an der Brust angelegten Grundzustand z.B. als flaches Gebilde, das keine glockenartige Struktur aufweist ausgebildet ist. Erst bei Anlegen an die Brust bildet sich die glockenartige Struktur instantan aus. Bei Entfernung von der Brust kehrt das Element wieder in seinen Grundzustand zurück.

Gemäß einer vorteilhaften Weiterbildung der Erfindung kann die Innenoberfläche der glockenartigen Struktur die Brust konturgenau abbilden und im Bereich der Brustwarze eine Ausnehmung bilden. Durch die konturgenaue Anlage ist es möglich, die Dekontaminationseinheit als einzelnes Element an einer wohldefinierten Stelle und in direkter Nähe zu dem zu behandelnden Bereich der Brust zu platzieren, ohne dass Bereiche auf der Brustoberfläche die nicht dekontaminiert werden sollen mittels der energetischen Strahlung bzw. Teilchen beaufschlagt werden .

Gemäß einer weiteren vorteilhaften Weiterbildung der Erfindung kann die Dekontaminationseinheit nach Montage der Vorrichtung an der Brust anliegen. Die Dekontaminationseinheit kann so vorzugsweise eine Fläche aufweisen, die unmittelbar an der zu behandelnden Oberfläche anliegt. Soweit die glockenartige Struktur im Bereich der Brustwarze an der Innenseite eine Ausnehmung bildet, kann die Innenoberfläche insbesondere die gesamte Innenoberfläche dieser Ausnehmung zum Beispiel durch die Dekontaminationseinheit gebildet werden. Alternativ können auch in dieser Ausnehmung eine Mehrzahl einzelner Dekontaminationseinheiten vorgesehen sein, jeweils für sich an der Brustwarze anliegend ausgebildet sind.

Gemäß einer vorteilhaften Weiterbildung der Erfindung können zumindest zwei Dekontaminationseinheiten an verschiedenen Positionen vorgesehen sein. So können eine oder mehrere Dekontaminationseinheit/en in der Ausnehmung und/oder auch eine oder mehrere Dekontaminationseinheiten in der glockenartigen Struktur angebracht sein. Diese sind zum Beispiel so angeordnet, dass der Warzenhof dekontaminiert werden kann. Eine oder mehrere Dekontaminationseinheiten können so angeordnet sein, dass diese oder eine Gruppe dieser die unterschiedlichen Regionen der Brust, nämlich die Brustwarze, der Warzenhof oder eine sonstigen Brustoberfläche selektiv dekontaminieren. Diese Dekontaminationseinheiten können untereinander unterschiedliche Intensitäten, beziehungsweise Stärken von Dekontaminationsintensität aufweisen, welche auf die unterschiedlichen Bereiche abgestimmt sind. So können unterschiedliche Bereiche der Brust selektiv dekontaminiert werden.

Gemäß einer vorteilhaften Weiterbildung der Erfindung kann die Dekontaminationseinheit beziehungsweise die Dekontaminationseinheiten derart positioniert sein, dass diese bei Montage der Vorrichtung an der Brust die Brustwarze physikalisch dekontaminieren. Zudem kann diese bei Montage der Vorrichtung an der Brust zusätzlich auch den Warzenhof dekontaminieren. Wie bereits beschrieben, können in diesem Fall die unterschiedlichen Dekontaminationseinheiten soweit sie beispielsweise die Brustwarze oder den Warzenhof dekontaminieren eine unterschiedliche Dekontaminationsintensität haben. Eine solche unterschiedliche Dekontaminationsintensität kann über die Wellenlänge der Lichtstrahlung, soweit es sich um eine Lichtquelle handelt, die Energie einer Ultraschallquelle, oder die Menge an generiertem Ozon beziehungsweise Plasma eingestellt werden.

Gemäß einer vorteilhaften Weiterbildung der Erfindung kann es sich bei der tragbaren Vorrichtung um einen Büstenhalter, oder ein Einlagepad, insbesondere Einlegekissen, oder eine elektrisch betriebene oder handbetriebene Milchpumpe handeln. Eine tragbare Vorrichtung ist eine Vorrichtung, die so leicht getragen werden kann, dass sie von einer einzigen Person ohne großen Aufwand und unter verschiedenen Umständen und an verschiedenen Orten verwendet werden kann. Sie kann besonders kompakt ausgebildet sein. Die gesamte Vorrichtung überschreitet vorzugsweise nicht das Gewicht von zwei Kilogramm, vorzugsweise einem Kilogramm. Soweit es sich um einen Büstenhalter handelt, kommen alle möglichen bekannten Arten von Büstenhaltern in Betracht. Milchpumpen werden zum Beispiel verwendet, um Milch von der weiblichen Brust abzupumpen.

Gemäß einer vorteilhaften Weiterbildung der Erfindung kann die Dekontaminationseinheit an eine autonome Energieversorgung angeschlossen sein, welche in der Vorrichtung integriert ist. Soweit es sich bei der Vorrichtung demnach um einen Büstenhalter, einer handbetriebenen oder elektrisch betriebenen Milchpumpe handelt, kann die Energieversorgung in der jeweiligen Vorrichtung integriert sein. Eine solche Energieversorgung kann eine wiederaufladbare Batterie oder ein Akkumulator sein. Eine solche Energieversorgung kann jedoch auch ein Transformator sein, der die Netzspannung in eine niedrigere Energieversorgungsspannung umwandelt. Vorzugsweise ist die Energieversorgung mit der Energieerzeugung kombiniert, das heißt, es handelt sich um eine wiederaufladbare Batterie oder um einen wiederaufladbaren Akkumulator.

Gemäß einer bevorzugten Weiterbildung der Erfindung kann die Dekontaminationseinheit in einem Pad bzw. Kissen vorgesehen sein, welches als separates Element in einen Büstenhalter einlegbar ist oder die Dekontaminationseinheit kann fest an der Vorrichtung montiert sein. Ein Kissen wird als schwammartig verformbares softes Pad verstanden. Soweit es sich um ein Pad handelt, in dem die Dekontaminationseinheit integriert ist, kann dieses beispielsweise in in dem Büstenhalter vorgesehene Taschen eingesetzt werden. Das Pad selbst weist dementsprechend die physikalische Dekontaminationseinheit auf. Das Pad kann eine separate autonome Einheit bilden, soweit die Energieversorgung und Energieerzeugung darin integriert ist. Im Falle der Verwendung der Vorrichtung in einer Milchpumpe kann die Dekontaminationseinheit vorzugsweise fest mit dieser verbunden sein. Dann ist gewährleistet, dass die Dekontaminationseinheit in vorbestimmter Anordnung zur Brust ausgerichtet ist, zum Beispiel wenn die Dekontaminationseinheit rückseitig innerhalb der glockenartigen Struktur, die einen Saugstutzen für die Brust bildet, angebracht ist.

Gemäß einer bevorzugten Weiterbildung der Erfindung können jeweils zumindest eine Dekontaminationseinheit auf einer der Brust zugewandten Seite und auf einer der Brust abgewandten Seite der Vorrichtung angeordnet sein.

Gemäß einer vorteilhaften Weiterbildung der Erfindung kann die Vorrichtung eine Steuereinrichtung aufweisen, welche die Dekontaminationseinheit ein- und ausschaltet und/oder die Intensität reguliert. So kann in der Vorrichtung eine Steuereinrichtung integriert sein, mittels welcher die Dekontaminationseinheit oder beziehungsweise die Dekontaminationseinheiten gesteuert werden. Eine solche Steuereinrichtung kann ein simpler An-/Ausschalter sein, der zwischen der Spannungsversorgung der Dekontaminationseinheit vorgesehen ist oder aber ein Mikrocomputer der in Abhängigkeit der Notwendigkeit nach Anbringen der Vorrichtung an der Brust die Dekontaminationseinheit beziehungsweise die Dekontaminationseinheiten steuert. So kann beispielsweise mittels eines Timers bestimmt werden wann die Pumpe angesetzt wurde, und daraus bestimmt werden wann die Dekontaminationseinheit eingeschaltet werden soll. Diese Steuereinrichtung kann z.B., soweit mehrere Dekontaminationseinheiten vorgesehen sind, diese selektiv steuern und/oder die Intensität der Dekontaminationsleistung in Abhängigkeit der Region wo die Dekontaminationseinheit an der Vorrichtung vorgesehen sind, variieren.

Gemäß einem nebengeordneten Aspekt nach Anspruch 12 betrifft die Erfindung auch den Betrieb einer tragbaren Vorrichtung zur Dekontamination einer Brust, wie sie zuvor beschrieben ist. Das Verfahren zeichnet sich dadurch aus, dass die Dekontaminationseinheit zur Abtötung Mikroorganismen eingeschaltet wird, um zu verhindern, dass diese in die Milchgänge der Brust eintreten.. Vorteilhafterweise wird der Betrieb der Vorrichtung nach Montage dieser Vorrichtung an der Brust eines weiblichen Menschen durchgeführt.

Weitere vorteilhafte Ausgestaltungen der Erfindung ergeben sich aus den nachfolgend erläuterten Ausführungsbeispielen in Verbindung mit der Zeichnung. In dieser zeigen:
- Figur 1: ein erstes Ausführungsbeispiel einer handbetriebenen Milchpumpe mit einer Dekontaminationseinheit,
- Figur 2a,2b, 2c: ein zweites Ausführungsbeispiel einer elektrisch betriebenen Milchpumpe,
- Figur 3a: ein drittes Ausführungsbeispiel, wobei der Aufsatz für eine Brust als glockenförmiger Aufsatz ausgebildet ist,
- Figuren 3b und 3c: eine Querschnittsansicht des Ausführungsbeispiel aus Figur 3a,
- Figuren 4a und 4b: ein in einen Büstenhalter eingelegtes Pad bzw. Kissen in dessen Querschnittsansicht,
- Figur 5: eine Spezialvorrichtung, die lediglich zur Dekontamination der Brust verwendet wird, sowie
- Figur 6: ein weiteres Beispiel einer alternativen Spezialvorrichtung, die lediglich zur Dekontamination der Brust verwendet wird.

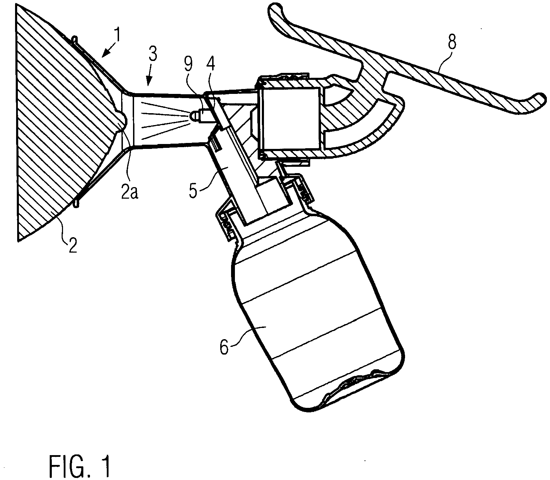

Die in Figur 1 dargestellte handbetriebene Milchpumpe ist ein Beispiel einer tragbaren Vorrichtung zur Dekontamination einer Brust 2. Diese handbetriebene Milchpumpe weist eine glockenartige Struktur 1 auf, die einen Bereich der Brust 2 dichtend umschließt, so dass aus dem Milchkanal der Brust 2 Milch herausgesogen werden kann. Die Brustwarze 2a ist in einem Bereich der glockenartigen Struktur 1 positioniert, wo diese eine konische, rohrartige Verjüngung 3 aufweist. Die konische, rohrartige Verjüngung 3 weist an deren der Brustwarze 2a gegenüber liegenden Seite 4 eine Leitung 5 auf, die zu einem Auffangbehälter 6 führt. Die glockenartige Struktur 1 ist integral mit der Leitung 5 und einem Schraubdeckel 7 verbunden und bildet somit eine Aufsatzeinheit, welche auf den Auffangbehälter 6 aufgeschraubt werden kann. Zudem ist an dieser Aufsatzeinheit ein Hebel 8 vorgesehen, über welchen ein Vakuum in der glockenartigen Struktur 1 und in dem Auffangbehälter 6 generiert werden kann.

An einer Wand an der der Brustwarze 2a gegenüber liegenden Seite 4 der konischen, rohrartigen Verjüngung 3 ist eine Dekontaminationseinheit 9 vorgesehen. Diese ist im vorliegenden Fall eine UV-Leuchtdiode (UV-LED) deren UV Licht auf die Brustwarze 2a und zumindest teilweise auf den um die Brustwarze herum ausgebildeten Warzenhof 10 abstrahlt.

Die UV-Leuchtdiode wird über ein in Figur 1 nicht dargestellten in die Aufsatzeinheit einsetzbaren Akku, welcher ein Standardmaß aufweist, mit Energie versorgt. In der Aufsatzeinheit befindet sich zudem eine nicht gezeigte Steuereinrichtung über welche die UV-Leuchtdiode an- und ausgestellt, und/oder reguliert werden kann. Alternativ zu der Stromversorgung mittels eines Akkumulators ist eine Stromversorgung mittels einer Batterie (nicht wiederaufladbar) denkbar. Alternativ kann auch ein Kondensator durch Betätigung des Hebels 8 aufgeladen werden der die Stromversorgung für die UV-Leuchtdiode bereitstellt. Es kann desweiteren in der Aufsatzeinheit eine Steuervorrichtung vorgesehen sein, die den Betrieb der Leuchtdiode derart steuert, dass diese beim Milchabpumpen oder lediglich kurz nach dem das Milchabpumpen beendet ist, angestellt wird. Auch kann die glockenartige Struktur 1 einen Kontaktsensor aufweisen, so dass die Leuchtdiode lediglich dann arbeitet, wenn die glockenartige Struktur 1 an der Brust anliegt. Diese Ausgestaltung bietet eine erhöhte Sicherheit, insbesondere wenn es sich um eine Dekontaminationsvorrichtung auf UV-Basis handelt, da so verhindert wird, dass ein Betrieb stattfindet, wenn eine Person in die Dekontaminationseinheit hereinschauen kann.

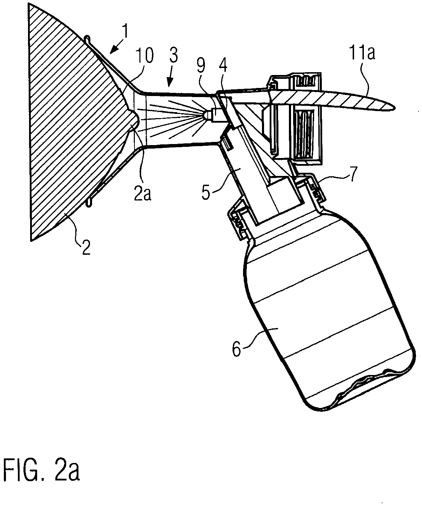

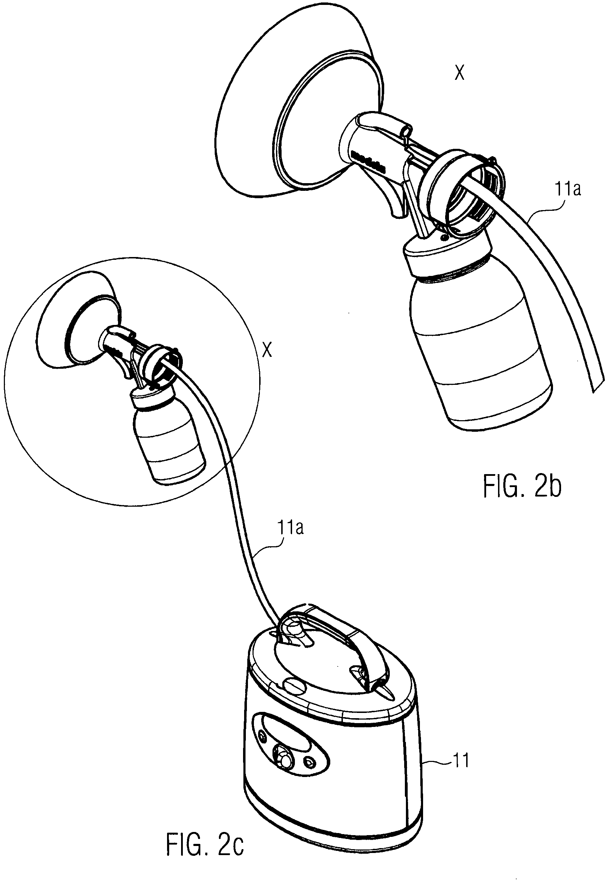

Ein alternatives Beispiel einer Milchpumpe ist in Figuren 2a, 2b und 2c gezeigt. Die Pumpe selbst ist gleich aufgebaut, wie die Pumpe in Figur 1, deshalb wird auf eine erneute Beschreibung der einzelnen Elemente verzichtet. Gleiche Elemente sind mit den gleichen Bezugszeichen versehen.
Im Unterschied zu der Pumpe aus Figur 1, handelt es sich bei dem Ausführungsbeispiel in Figuren 2a, 2b und 2c nicht um eine handbetriebene Pumpe sondern um eine elektrisch betriebene Pumpe.

Wie in Figuren 2a und 2b zu erkennen ist eine Vakuumerzeugungsstation 11 vorgesehen, die separat von der Aufsatzeinheit und dem Auffangbehälter 6 vorgesehen ist. Die Aufsatzeinheit mit dem Auffangbehälter 6 ist über den Vakuumschlauch 11a an die Vakuumerzeugungsstation 11 angebunden. Eine Querschnittsansicht der Aufsatzeinheit und dem Auffangbehälter 6 ist in Figur 2a zu sehen. Wie daraus zu erkennen, ist das Innere der glockenartigen Struktur 1 gleich ausgebildet, wie im Ausführungsbeispiel nach Figur 1.

Bei der handbetriebenen Pumpe aus Figur 1 sollte eine unabhängige separate Energieversorgungsquelle in der Vorrichtung vorgesehen sein. Bei der elektrisch betriebenen Pumpe aus Figuren 2a, 2b und 2c kann, entweder eine separate Energieerzeugung zusätzlich zu der Energieversorgung der Vakuumerzeugungsstation 11 vorgesehen sein und/oder die Dekontaminationseinheit 9 wird über die Vakuumerzeugungsstation 11 mit Spannung versorgt.

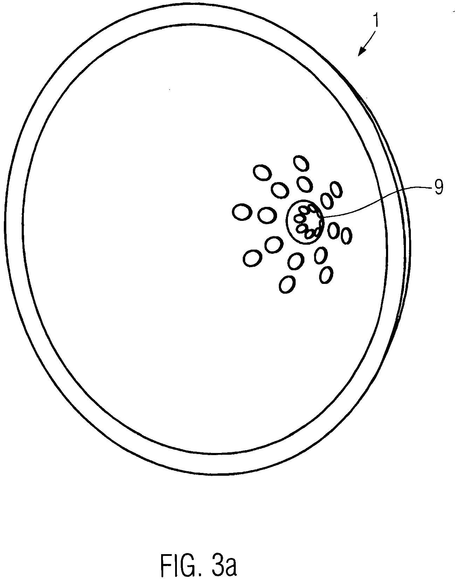

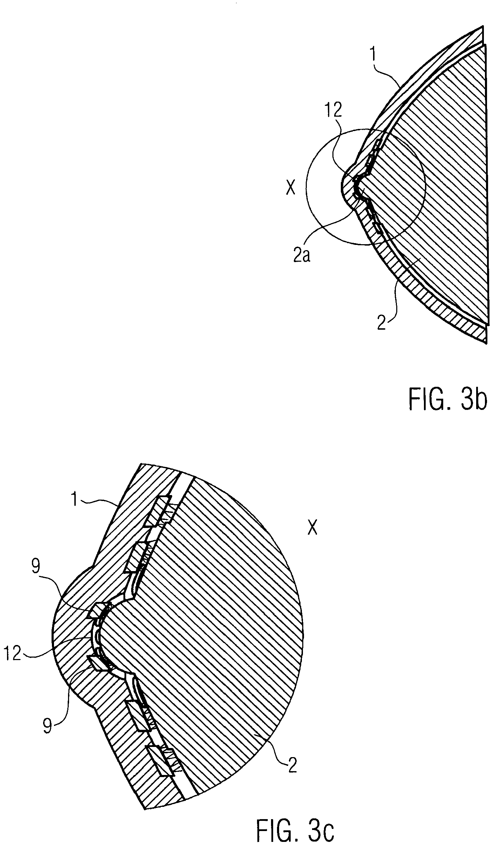

Figur 3a zeigt eine alternative Ausgestaltung einer glockenartigen Struktur 1. Die glockenartige Struktur 1 ist in diesem Fall schon so vorgeformt, dass diese an die Brust anlegbar ist. Alternativ kann diese auch so ausgebildet sein, das sie in einem nicht an der Brust angelegten Grundzustand als flaches Gebilde, das keine glockenartige Struktur aufweist ausgebildet ist. Erst bei Anlegen an die Brust 2 bildet sich die glockenartige Struktur 1 instantan aus. Bei Entfernung von der Brust 2 kehrt das Element wieder in seinen Grundzustand zurück. Wie in der Querschnittsansicht in Figur 3b und 3c zu sehen ist, ist diese glockenartige Struktur 1 derart ausgebildet, dass diese konturabbildend an der Brust 2 anliegt. Hierzu weist die glockenartige Struktur 1 im Bereich der Brustwarze 2a eine Ausnehmung 12 auf. Innerhalb der Ausnehmung 12 sind in einer kreisförmigen Anordnung einzelne Dekontaminationseinheiten 9 angebracht. In der glockenartigen Struktur 1 außerhalb der Ausnehmung 12 sind im Bereich wo die glockenartige Struktur 1 an den Warzenhof anliegt mit einem geringen Abstand zum Warzenhof weitere Dekontaminationseinheiten 9 vorgesehen. Alle Dekontaminationseinheiten 9 können über eine gemeinsame, nicht dargestellte, Steuereinrichtung angesteuert werden. Diese Steuereinrichtung, sowie auch eine Energieversorgung der Dekontaminationseinheiten kann in der glockenartigen Struktur 1 integriert vorgesehen sein.

Wie in Figuren 3a und 3b dargestellt, haben die Dekontaminationseinheiten 9 im Bereich der Brustwarze eine leicht gerundete Oberfläche um planar und im Wesentlichen vollflächig an der gerundeten Brustwarze 2a anzuliegen. Diese außerhalb der Ausnehmung 12 vorgesehenen Dekontaminationseinheiten 9 haben eine planare Fläche.

Die bei zentraler Aufsicht auf die glockenartige Struktur 1 radial am weitesten außenliegenden Dekontaminationseinheiten 9 sind an der Grenze zwischen Warzenhof und normaler Brusthaut angeordnet.

Die Dekontaminationseinheiten aus den Figuren 3a bis 3c können flächige Lichtquellen sein oder auch piezokeramische Elemente, die Ultraschallwellen ausstrahlen.

Auch wenn vorliegend für das Ausführungsbeispiel aus Figur 3 eine glockenartige Struktur 1 vorgesehen ist, kann eine ähnliche Anordnung auch innerhalb eines separaten Pads welches in einen Büstenhalter, z.B. in eine Tasche eines Büstenhalters eingelegt wird, vorgesehen sein.

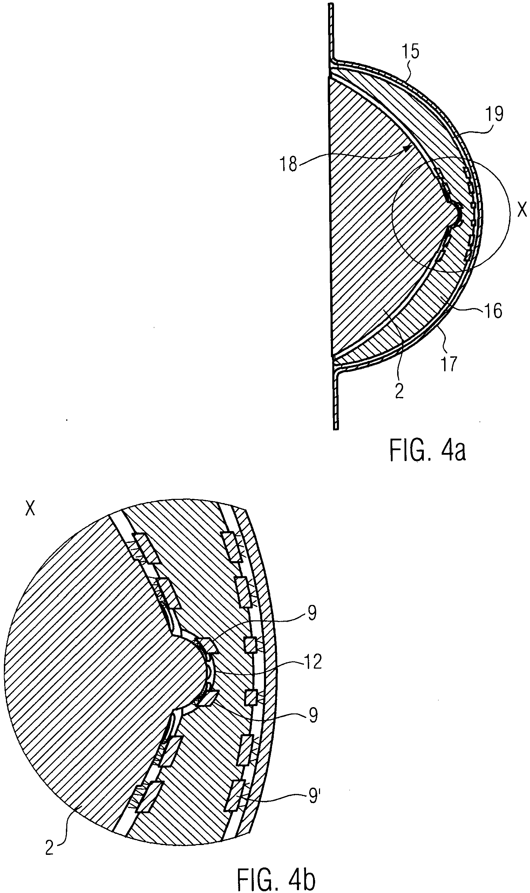

Ein solches in einen Büstenhalter 15 eingelegtes Pad 16, ist in Figuren 4a und 4b gezeigt. Hierbei ist Figur 4b der Ausschnitt X aus Figur 4a. Das Pad 16 ist in ein Körbchen 17 des Büstenhalters 15 eingelegt. Ein solches Pad16 kann aus einem kissenartigen sich schwammartig verformenden Material sein. In einem nicht in den Büstenhalter 15 eingesetzten Grundzustand kann das Pad 16 als flaches kissenartiges Gebilde (z.B. mit zwei korrespondierenden Oberflächenwölbungen auf entgegengesetzten Seiten), das keine glockenartige Struktur aufweist ausgebildet sein. Erst bei Einlegen in den Büstenhalter 15 und Anlage an der Brust bildet sich die glockenartige Struktur 1 instantan aus. Bei Entfernung aus dem Büstenhalter kehrt das Pad wieder in seinen Grundzustand zurück. Auf dessen der Brust 2 zugewandten Seite 18 ist das Pad so aufgebaut wie die glockenartige Struktur 1 in Figuren 3 a bis c, deshalb wird auf eine erneute Beschreibung der einzelnen Elemente verzichtet. Gleiche Elemente sind mit den gleichen Bezugszeichen versehen. Zusätzlich sind aber auch Dekontaminationseinheiten 9' an der der Brust abgewandten Seite 19 des Pads 16 vorgesehen.

Auch die Dekontaminationseinheiten 9' an der der Brust abgewandten Seite 19 des Pads 16 eine leicht gerundete Oberfläche um planar an der Innenseite des Kröbchens 17 anzuliegen. Die Dekontaminationseinheiten 9' an der der Brust abgewandten Seite 19 des Pads 16 sind vorteilhaft, um auch das Körbchen 17 des Büstenhalter 15 zu dekontaminieren.

Die glockenartige Struktur 1 im Ausführungsbeispiel in Figuren 3a bis 3c kann aus Silikon oder aus Kunststoff oder auch aus einem Schaumstoffmaterial sein.

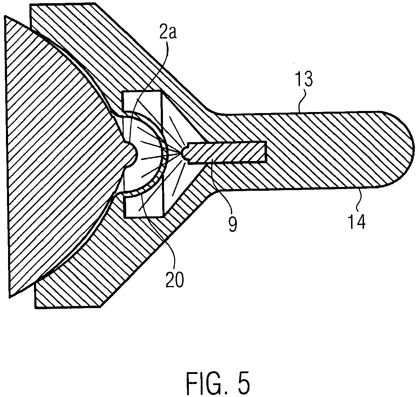

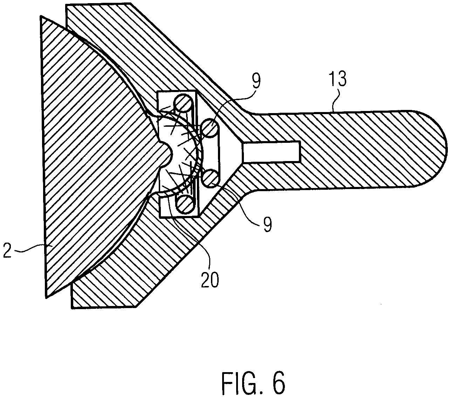

Figuren 5 und 6 zeigen zwei Ausführungsbeispiele, wobei die Vorrichtung zur Dekontamination in diesem Fall eine Vorrichtung ist, die lediglich die Funktionalität der Dekontamination hat. Das heißt, diese Vorrichtung weist nicht gleichzeitig die Funktion eines Büstenhalters, beziehungsweise die Funktion einer Milchpumpe auf.

Die Vorrichtung ist als eine Art Stempel 13, ausgebildet mit einem rückseitig vorgesehen Griffteil 14. Der Stempel 13 weist eine glockenartige Innenoberfläche auf, welche auf die Brust gestülpt wird.

Wie in Figur 5 zu sehen, ist bei dieser Ausführungsform auch ein UV-Lichtelement als Dekontaminationselement 9 gegenüber der Brustwarze 2a positioniert.

Das Ausführungsbeispiel aus Figur 6 unterscheidet sich von dem Ausführungsbeispiel aus Figur 5 dadurch, dass anstelle dieses einzigen Dekontaminationselements 9, welches gegenüber der Brustwarze ausgebildet ist, eine Mehrzahl an Dekontaminationselementen 9 vorgesehen ist, die die Brustwarze von verschiedenen Seiten behandeln.

In beiden Ausführungsbeispielen ist eine Separationswand 20 vorgesehen, welche die Dekontaminationseinheit 9, von einem Raum 21, der zwischen der Separationswand 20 und der Brustwarze 2a gebildet ist separiert. So ist die Innenoberfläche des Stempels 13 leichter zu reinigen, und die Dekontaminationseinheit 9 kommt nicht unmittelbar mit der Brust in Kontakt. Soweit es sich bei der Dekontaminationseinheit 9 um eine UV-Lampe handelt, sollte die Separationswand 20 UV-transparent sein.

### Bezugszeichenliste

glockenartige Struktur 1
Brust 2
Brustwarze 2a
konische rohrartige Verjüngung 3
der Brustwarzen gegenüberliegende Seite 4
Leitung 5
Auffangbehälter 6
Schraubdeckel 7
Hebel 8
Dekontaminationseinheit 9, 9'
Warzenhof 10
Vakuumerzeugungsstation 11
Vakuumschlauch 11a
Ausnehmung 12
Stempel 13
Griffteil 14
Büstenhalter 15
Pad 16
Körbchen 17
der Brust zugewandte Seite 18
der Brust abgewandte Seite 19
Separationswand 20
Raum 21

## Patentansprüche

1. Tragbare Vorrichtung zur Dekontamination einer Brust (2)
**gekennzeichnet durch**
eine Dekontaminationseinheit (9), welche die Brust (2) zumindest mittels einer physikalischen Methode dekontaminiert.

2. Vorrichtung nach Anspruch 1, **dadurch gekennzeichnet, dass** die Dekontaminationseinheit (9) zumindest eines der folgenden Elemente enthält: eine Lichtquelle, eine Ultraschallquelle, eine Ozonisierungsquelle, eine Plasmaquelle.

3. Vorrichtung nach einem der vorherigen Ansprüche, **gekennzeichnet, durch** eine glockenartige Struktur (1) vorgesehen ist, welche über die Brust (2) stülpbar ist und diese zumindest bereichsweise umschließt.

4. Vorrichtung nach Anspruch 3, **dadurch gekennzeichnet, dass** die glockenartige Struktur (1) die Brust (2) konturgenau abbildet und im Bereich der Brustwarze (2a) an deren Innenseite eine Ausnehmung (12) bildet.

5. Vorrichtung nach Anspruch 4, **dadurch gekennzeichnet, dass** die glockenartige Struktur 1 so ausgebildet ist, das sich diese erst bei Anlegen an die Brust (2) instantan ausbildet und vorzugsweise bei Entfernung von der Brust (2) wieder in deren Grundzustand zurückkehrt.

6. Vorrichtung nach einem der vorherigen Ansprüche 1, **dadurch gekennzeichnet, dass** die Dekontaminationseinheit (9) nach Montage der Vorrichtung an der Brust (2), an der Brust (2) anliegt.

7. Vorrichtung nach einem der vorherigen Ansprüche, **dadurch gekennzeichnet, dass** zumindest zwei Dekontaminationseinheiten (9) an verschiedenen Positionen vorgesehen sind.

8. Vorrichtung nach einem der vorherigen Ansprüche, **dadurch gekennzeichnet, dass** die Dekontaminationseinheit (9) bzw., die Dekontaminationseinheiten (9) derart positioniert sind, dass diese bei Montage der Vorrichtung an der Brust (2) die Brustwarze (2a) dekontaminiert.

9. Vorrichtung nach einem der vorherigen Ansprüche, **dadurch gekennzeichnet, dass** es sich bei der Vorrichtung um einen Büstenhalter, oder ein Einlagepad, insbesondere Einlegekissen, oder eine elektrisch betriebene oder handbetriebene Milchpumpe handelt, wobei die Dekontaminationseinheit in darin integriert ist.

10. Vorrichtung nach einem der vorherigen Ansprüche, **dadurch gekennzeichnet, dass** die Dekontaminationseinheit (9) an eine autonome Energieversorgung angeschlossen ist, welche in der Vorrichtung integriert vorgesehen ist.

11. Vorrichtung nach einem der vorherigen Ansprüche, **dadurch gekennzeichnet, dass** die Dekontaminationseinheit (9) in einem Pad bzw. Kissen vorgesehen ist, welches als separates Element in einen Büstenhalter einlegbar ist oder, dass die Dekontaminationseinheit (9) fest an der Vorrichtung montiert ist.

12. Vorrichtung nach einem der vorherigen Ansprüche, **dadurch gekennzeichnet, dass** jeweils zumindest eine Dekontaminationseinheit (9, 9') auf einer der Brust (2) zugewandten Seite (20) und auf einer der Brust (2) abgewandten Seite (19) der Vorrichtung angeordnet ist.

13. Vorrichtung nach einem der vorherigen Ansprüche, **dadurch gekennzeichnet, dass** diese eine Steuereinrichtung aufweist, welche die Dekontaminationseinheit (9) ein und/oder ausschaltet, und/oder die Intensität reguliert.

14. Betrieb einer tragbaren Vorrichtung zur Dekontamination einer Brust (2) nach einem der vorherigen Ansprüche, **dadurch gekennzeichnet, dass** die Dekontaminationseinheit (9) zur Abtötung von Mikroorganismen eingeschaltet wird, um zu verhindern, dass diese in die Milchgänge der Brust (2) eintreten.
